# EUROPÄISCHE PATENTSCHRIFT

(11) **EP 1 568 329 B1**
(45) Veröffentlichungstag und Bekanntmachung des Hinweises auf die Patenterteilung: **20.06.2007**
(21) Anmeldenummer: 05002783.8
(22) Anmeldetag: 10.02.2005
(51) Int. Cl.: A61B 17/86, A61B 17/68

(54) **Knochenverankerungselement**
Bone-anchoring element
Elément d'ancrage osseux

(30) Priorität: 24.02.2004 DE 102004009429; 24.02.2004 US 547517 P
(43) Veröffentlichungstag der Anmeldung: 31.08.2005
(73) Patentinhaber: BIEDERMANN MOTECH GmbH, 78054 Villingen-Schwenningen (DE)
(72) Erfinder: Biedermann, Lutz, 78084 VS-Villingen (DE); Matthis, Wilfried, 79367 Weisweil (DE); Rapp, Helmar, 78652 Deisslingen (DE)
(74) Vertreter: Hofer, Dorothea

(56) Entgegenhaltungen:
- EP-A- 0 340 159
- EP-A- 0 714 643
- CH-A- 682 450
- DE-A1- 19 731 298
- DE-A1- 19 801 219
- DE-U1- 29 914 192
- US-A- 5 196 016
- US-A- 5 562 704

## Beschreibung

Die Erfindung betrifft ein Knochenverankerungselement.

Eine bekannte Form eines Knochenverankerungselements ist eine Knochenschraube, die einen Schaft mit einem Gewinde zum Einschrauben in einen Knochen aufweist. Das Einbringen in den Knochen erfolgt durch manuelles Einschrauben mittels eines Schraubendrehers, was ein zeitaufwendiger und krafterfordernder Vorgang ist. Zudem können beim Einschrauben hohe Druckkräfte auf die Knochenstruktur ausgeübt werden, was in manchen Fällen nicht wünschenswert ist. Deshalb ist für bestimmte klinische Anforderungen, insbesondere in der Neurochirurgie, der Wirbelsäulenchirurgie oder der Kinderchirurgie das manuelle Einschrauben von Knochenverankerungselementen weniger geeignet.

Aus der EP 0 714 643 A1 ist ein Knochenverankerungselement mit einem Schaft und einem Knochengewinde bekannt, wobei auf den Gewindeflanken des Knochengewindes eine Mikrotextur vorgesehen ist, die Elemente aufweist, die widerhakenähnliche Funktionen haben. Dadurch ist das Einschrauben leicht möglich, das Herausschrauben jedoch behindert. In einem weiteren Beispiel sind stachelartige Spitzen als Mikrorauhigkeit an einem Knochenstift vorgesehen.

Aus der DE 198 01 219 A1 ist ein Knochennagel bekannt, der an seinem Schaft eine Mehrzahl von Vorsprüngen aufweist, die ein einfachen Eintreiben des Nagels ermöglichen, jedoch ein Herausziehen des Nagels behindern.

Die CH 682450 A5 offenbart ein Knochenverankerungselement nach dem Oberbegriff des Patentanspruch 1.

Es ist Aufgabe der vorliegenden Erfindung, ein Knochenverankerungselement bereitzustellen, das schneller, einfacher und mit weniger Kraftaufwand in den Knochen einzubringen ist, keine schädigenden Kräfte auf den Knochen ausübt, einen sicheren Halt gewährleistet und dennoch im Sinne einer Schraube tiefer eingebracht bzw. entfernt werden kann.

Die Aufgabe wird gelöst durch ein Knochenverankerungselement nach Patentanspruch 1. Weiterbildungen der Erfindung sind in den Unteransprüchen angegeben.

Das Knochenverankerungselement weist den Vorteil auf, daß eine schnelle und sichere Verankerung durch Eindrücken des Verankerungselements in ein vorgebohrtes Kernloch im Knochen möglich ist. Durch die Anordnung der Widerhakenelemente auf einer Schraubenlinie ist eine gewindeähnliche Funktion gegeben, die eine zusätzliche Tiefenpositionierung durch eine Einschraubbewegung ähnlich der von Knochenschrauben erlaubt. Ferner ist durch die Widerhakenelemente eine Sicherheit gegen Herausziehen oder Lockerung des Knochenverankerungselements gegeben. Das eventuelle Entfernen des Knochenverankerungselements erfolgt wie bei einer Schraube durch Rückdrehung.

Weitere Merkmale und Zweckmäßigkeiten der Erfindung ergeben sich aus der Beschreibung von Ausführungsbeispielen anhand der Figuren. Von den Figuren zeigen:
- Fig. 1: eine perspektivische Ansicht eines Knochenverankerungselements nach einer ersten Ausführungsform;
- Fig. 2: eine perspektivische Explosionsdarstellung des Knochenverankerungselements von Fig. 1;
- Fig. 3: eine Schnittansicht des Knochenverankerungselements von Fig. 1 entlang der Linie III-III;
- Fig. 4: eine vergrößerte perspektivische Ansicht eines Details des Knochenverankerungselements von Fig. 1;
- Fig. 5: eine perspektivische Ansicht eines Elements des Knochenverankerungselements nach den Figuren 1 bis 4 in einem ersten Zustand;
- Fig. 6: eine perspektivische Ansicht des Elements gemäß Fig. 5 in einem zweiten Zustand;
- Fig. 7: eine Schnittansicht des Elements von Fig. 6 gemäß Linie II-II in Fig. 6;
- Fig. 8a): eine schematische Darstellung, die das Einbringen des Knochenverankerungselements nach den Figuren 1 bis 7 in einem ersten Schritt zeigt;
- Fig. 8b): eine schematische Darstellung, die das Einbringen eines Knochenverankerungselements nach einer abgewandelten Ausführungsform zeigt;
- Fig. 9a): eine schematische Darstellung, die das Einbringen einer abgewandelten Ausführungsform des Knochenverankerungselements nach Fig. 1 bis 7 in einem ersten Schritt zeigt;
- Fig. 9b): eine schematische Darstellung, die das Einbringen der abgewandelten Ausführungsform in einem zweiten Schritt zeigt;
- Fig. 10: eine perspektivische Ansicht eines Knochenverankerungselements nach einer weiteren Abwandlung der ersten Ausführungsform;
- Fig. 11a): eine Schnittansicht des Knochenverankerungselements nach Fig. 10 entlang der Linie IV-IV;
- Fig. 11b), 11c): alternative Ausführungsformen für den Querschnitt des Knochenverankerungselements;
- Fig. 12: eine perspektivische Ansicht eines Knochenverankerungselements nach einer weiteren Abwandlung der ersten Ausführungsform;
- Fig. 13a) bis 13c): Schritte der Herstellung eines Knochenverankerungselements nach einer zweiten Ausführungsform;
- Fig. 14: eine Ansicht im Längsschnitt durch das Knochenverankerungselement nach dem letzten Herstellungsschritt gemäß Fig. 13c);
- Fig. 15: eine Querschnittsansicht durch den Schaft des Knochenverankerungselements gemäß Fig. 13c) der zweiten Ausführungsform;
- Fig. 16 bis Fig. 23: Beispiele für die Verwendung des Knochenverankerungselements.

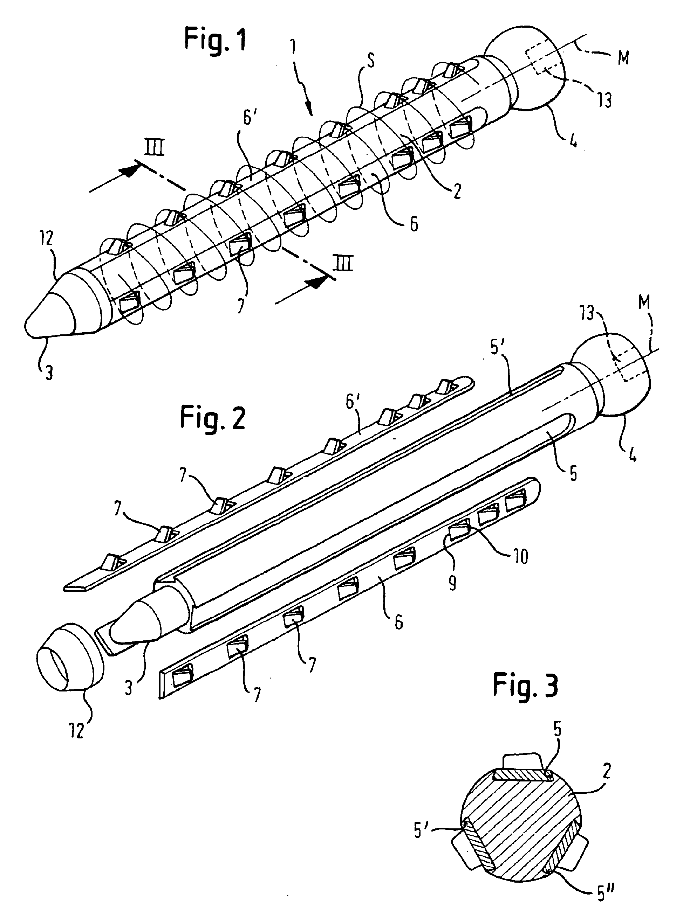

Im folgenden wird mit Bezug auf die Figuren 1 bis 7 ein Knochenverankerungselement nach einer ersten Ausführungsform beschrieben.

Das Knochenverankerungselement 1 weist einen Schaft 2 mit einer Spitze 3 an einem Ende und einem Kopf 4 an dem anderen Ende auf. Der Schaft 2 ist zylindrisch ausgebildet und weist eine Mehrzahl von sich parallel zur Schaftachse M erstreckende Nuten 5, 5' auf, die an ihrem der Spitze 3 zugewandten Ende offen sind und an ihrem dem Kopf 4 zugewandten Ende, bevorzugt mit einer Rundung, geschlossen sind. Das Profil der Nuten 5 ist, wie insbesondere aus Fig. 3 ersichtlich ist, schwalbenschwanzförmig, es kann aber auch eine andere Form, wie z.B. eine T-Form aufweisen. In dem dargestellten Beispiel sind drei in Umfangsrichtung des Schafts 2 gleichmäßig beabstandete Nuten 5 vorgesehen, es können jedoch auch wenigstens zwei oder mehr als drei Nuten vorgesehen sein.

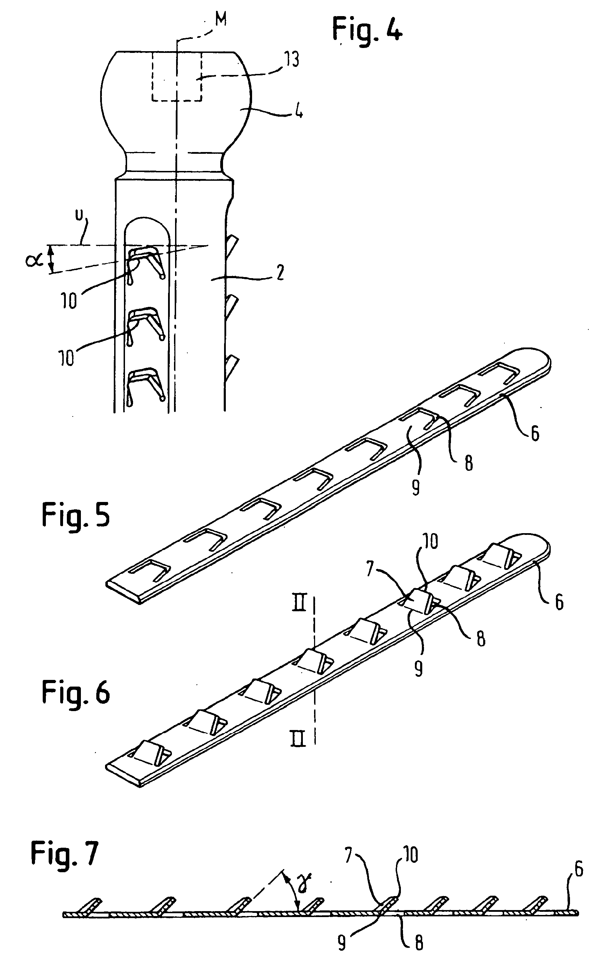

In die Nuten 5 sind längliche Bleche 6, 6' eingesetzt, deren Querschnitt dem der Nuten 5 angepaßt ist, so daß sie in die Nuten 5, 5' einschiebbar sind und darin paßförmig gehalten sind. Die länglichen Bleche 6 weisen jeweils eine Mehrzahl von Widerhakenelementen 7 auf. Die Widerhakenelemente 7 sind, wie insbesondere aus den Figuren 5 bis 7 ersichtlich ist, durch Bilden von im wesentlichen trapezförmigen Einschnitten 8 in den Blechen 6 gebildet, wobei die Basis 9 des Trapezes der Ansatz und die Biegungsstelle für die Widerhakenelemente 7 im Blech 6, 6' ist. Die Widerhakenelemente 7 sind so angeordnet, daß in dem in die Nuten 5 eingesetzten Zustand die breitere Basis 9 der Spitze 3 zugewandt ist und die schmäleren freien Enden 10 der Widerhakenelemente 7 dem Kopf 4 zugewandt sind.

Wie aus den Figuren 5 bis 7 ersichtlich ist, stehen die Widerhakenelemente 7 um einen vorbestimmten Winkel γ von der Oberfläche weg, der in Abhängigkeit von dem verwendeten Material und den Abmessungen der Widerhakenelemente 7 bei der Herstellung so gewählt wird, daß eine gewünschte Steifigkeit gegeben ist. Die Widerhakenelemente 7 sind aufgrund ihrer Anbindung nur über die Basis 9 elastisch und weisen in einem in die Einschnitte 8 eingedrückten Zustand eine Vorspannung auf.

Wie aus Fig. 1 ersichtlich ist, sind die Widerhakenelemente 7 eines Bleches 6 jeweils relativ zu den Widerhakenelementen 7 eines benachbarten Bleches 6' in axialer Richtung auf einer Schraubenlinie S um die Schraubenachse M angeordnet. Die im wesentlichen trapezförmigen Einschnitte 8 sind ferner, wie insbesondere aus der vergrößerten Darstellung gemäß Fig. 4 ersichtlich ist, unter einem Winkel α gegen die Kreisumfangslinie U geneigt, der dem Steigungswinkel der Schraubenlinie S entspricht. Die freien Enden 10 der Widerhakenelemente liegen somit auf der Schraubenlinie S, so daß sie Schneidkanten ähnlich der Spitze eines Schraubengewindes bilden.

Wie ferner aus den Figuren 1 und 2 ersichtlich ist, ist bei dem gezeigten Ausführungsbeispiel in einem kopfnahen Bereich in jeder Windung der imaginären Schraubenlinie S ein Widerhakenelement 7 vorgesehen, und in einem Bereich angrenzend an die Spitze 3 ist in jeder zweiten Windung ein Widerhakenelement vorgesehen.

Wie aus Fig. 2 ersichtlich ist, weist ferner die Spitze 3 in einem an den Schaft 2 angrenzenden Abschnitt einen kleineren Durchmesser auf als der Schaft 2, damit das Einschieben der Bleche 6 in die Nuten möglich ist. Im montierten Zustand der Bleche 6, in dem diese in die Nuten 5 eingeschoben sind, ist ferner ein ringförmiges Sicherungselement 12 vorgesehen, welches an seinem dem Schaft zugewandten Ende bündig mit dem Schaft 2 abschließt, so daß ein Herausschieben der Bleche 6 verhindert wird, und welches sich dann mit einem kegeligen Abschnitt zur Spitze 3 hin verjüngt und bündig an diese anschließt. Das Sicherungselement 12 ist fest mit der Spitze verbunden, bevorzugt angeschweißt.

Der Kopf 4 weist in dieser Ausführungsform eine Kugelsegmentform auf und hat an seiner dem Schaft abgewandten Seite eine Ausnehmung 13 zum Eingreifen mit einem Eindrehwerkzeug.

Das Verankerungselement ist aus einem körperfreundlichen Material, insbesondere aus einem körperfreundlichen Metall, wie beispielsweise Titan, oder aus einem körperfreundlichen Kunststoff gebildet. Die Bleche 6 sind aus demselben Material wie der Schaft gebildet oder aus einem anderen Material, wenn für die Bleche aufgrund der geforderten elastischen Eigenschaften der Widerhakenelemente 7 ein anderes Material benötigt wird.

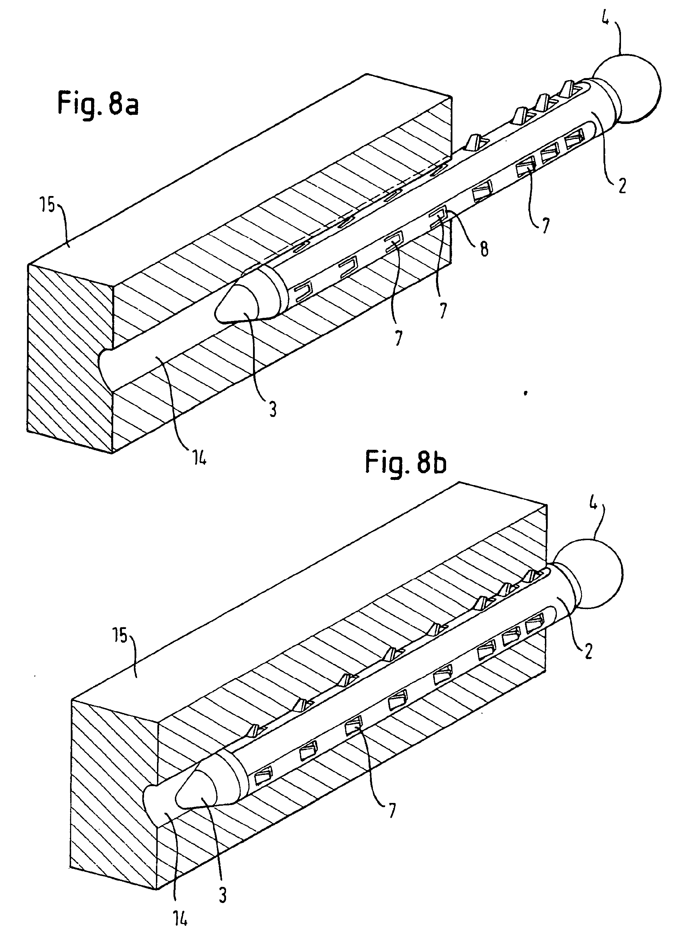

Im Betrieb wird das Verankerungselement 1 zunächst vormontiert, indem die vorgefertigten, mit den Widerhakenelementen 7 versehenen Bleche 6, 6' in die Nuten 5, 5' eingeführt werden, und dann das Sicherungselement 12 fest mit der Spitze verbunden wird. In der Anwendung wird zunächst, wie schematisch aus den Figuren 8a) und 8b) ersichtlich ist, ein Kernloch 14 in den Knochen 15 gebohrt. Der Durchmesser des Kernlochs entspricht im wesentlichen dem Durchmesser des Schafts 2 oder er ist geringfügig größer oder kleiner. Anschließend wird, wie insbesondere aus Fig. 8a) ersichtlich ist, das Verankerungselement 1 in das Kernloch 14 eingedrückt. Aufgrund ihrer Elastizität werden die Widerhakenelemente 7 beim Eindrücken gegen bzw. in die Einschnitte 8 gepreßt. Das Verankerungselement läßt sich durch die Gleitbewegung im Unterschied zum herkömmlichen Einschrauben sehr schnell einführen.

Im eingeführten Zustand stellen sich die unter Vorspannung stehenden Widerhakenelemente 7, wie aus Fig. 8b) ersichtlich ist, auf und drücken mit ihrer Schneidkante 10 nach außen gegen die Wandung des Kernlochs 14. Durch die Widerhakenwirkung wird ein Herausziehen verhindert.

Zum korrekten Positionieren des Verankerungselements in der Tiefe im Kernloch 14 bzw. der Stellung des Kopfs wird es durch Angreifen mit dem Eindrehwerkzeug am Kopf 4 wie eine Schraube durch eine Drehbewegung weiter in das Kernloch 14 hinein oder aus diesem herausgeschraubt. Die Schneidkanten 10 der Widerhakenelemente, die auf der Schraubenlinie S liegen, wirken dabei wie die Spitze eines Gewindes. Ein eventuelles Entfernen des Knochenverankerungselements erfolgt wie bei einer Schraube durch Rückdrehung.

Bei einer herkömmlichen Knochenschraube ist zusätzlich zum Bohren des Kernloches meistens das Schneiden des Knochengewindes in den Knochen und immer eine wiederholte Drehbewegung erforderlich. Im Vergleich zur Zeit, die zum Verankern einer herkömmlichen Knochenschraube benötigt wird, ist die Zeit, die zum Einbringen des Verankerungselements der Erfindung benötigt wird, aufgrund der Gleitbewegung beim Einschieben erheblich kleiner ohne auf die Vorteile eines Schraubengewindes beim Positionieren oder Herausdrehen zu verzichten.

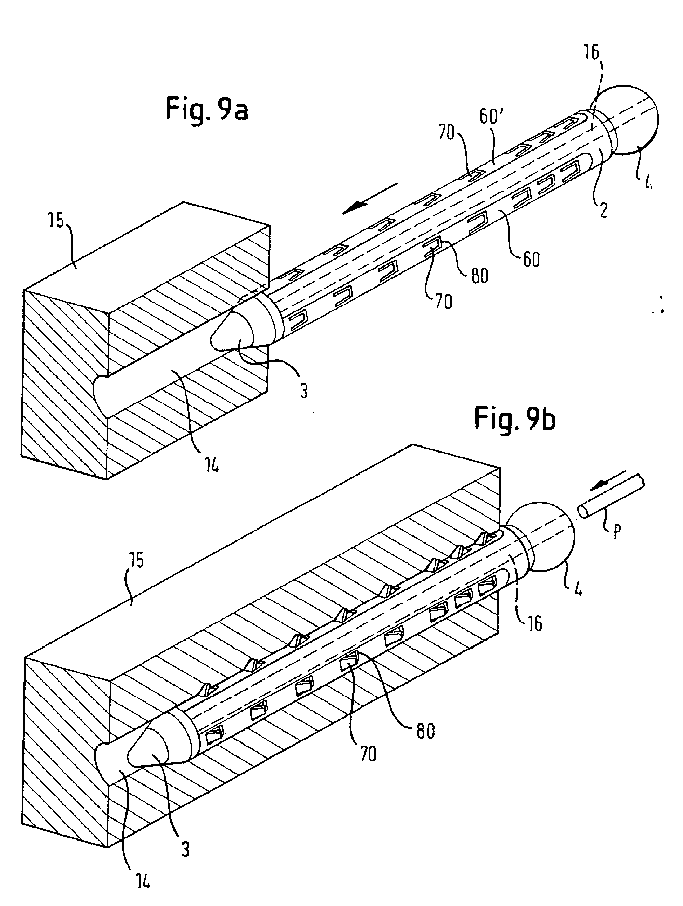

Fig. 9a) und 9b) zeigen den Aufbau und den Betrieb einer Abwandlung der ersten Ausführungsform.

Diese unterscheidet sich von der ersten Ausführungsform dadurch, daß die Bleche 60 mit den Widerhaken 70 aus einer körperfreundlichen Legierung mit Formgedächtnis- und/oder superelastischen Eigenschaften, beispielsweise einer Titannickellegierung, bevorzugt aus Nitinol bestehen. Die Bleche 60 werden dabei so gefertigt, daß die Widerhakenelemente 70 in einem Zustand mit einer vorbestimmten erhöhten Temperatur nach außen abstehend geformt sind, bei einer relativ zu dieser vorbestimmten erhöhten Temperatur niedrigeren Temperatur aber in den Ausschnitten 80 liegen. In diesem Zustand wird, wie in Fig. 9a) gezeigt ist, das Verankerungselement in das Kernloch 14 eingeschoben. Nach dem Einschieben wird wie in Fig. 9b gezeigt ist, mittels z.B. einer Heizpatrone P, die in eine dafür vorgesehene koaxiale Bohrung 16 im Inneren des Verankerungselements eingeschoben wird, das Verankerungselement auf die vorbestimmte Temperatur erwärmt, wobei sich durch den dabei stattfindenden Phasenübergang in der Kristallstruktur der Bleche die Widerhakenelemente 70 aufstellen und gegen die Wand des Kernlochs 14 drücken. Die Heizpatrone wird anschließend wieder entfernt.

Diese abgewandelte Ausführungsform hat den Vorteil, daß eine reduzierte Einpreßkraft erforderlich ist und eine Justierung der Tiefenposition in gewissem Umfang durch eine Gleitbewegung erfolgen kann, solange die Widerhakenelemente 70 noch nicht gegen die Wand des Kernlochs 14 drücken. Bei Vorliegen von Superelastizität weisen die Widerhakenelemente 70 aufgrund der Superelastizität der Legierung eine etwa zehnprozentige Dehnung auf, gegenüber einer etwa einprozentigen Dehnung von herkömmlichen Metallen. Diese größere Elastizität erleichtert die Handhabung und sichert zusätzlich die Verankerung im Knochen.

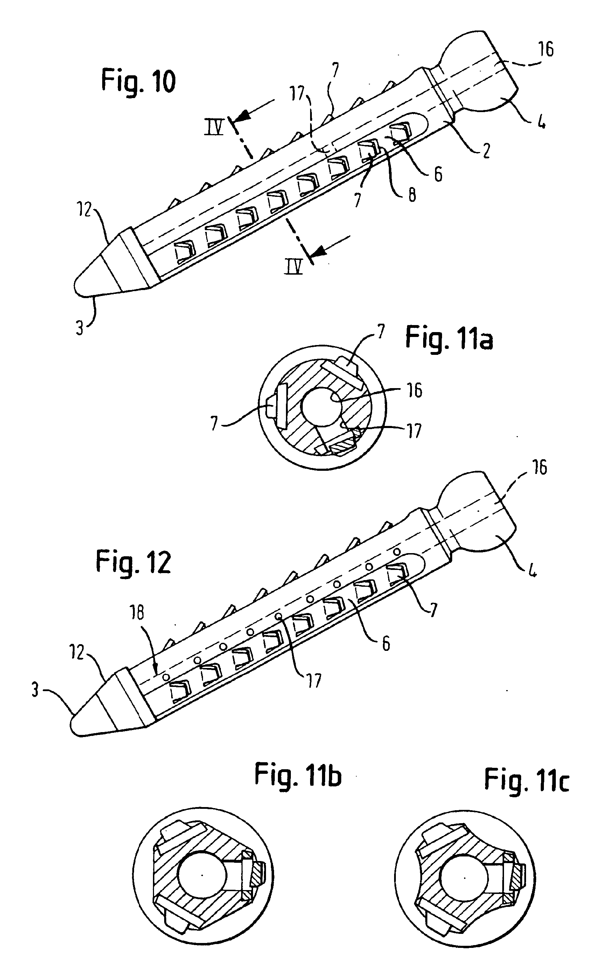

In einer weiteren in den Figuren 10 und 11a) gezeigten Abwandlung der ersten Ausführungsform weist das Verankerungselement die bereits bei der zuvor genannten Abwandlung beschriebene, sich vom freien Ende des Kopfs 4 bis zum Ende des Schafts 2 erstreckende koaxiale Bohrung 16 auf. Die koaxiale Bohrung 16 steht über in radial verlaufender Richtung vorgesehene Bohrungen 17 mit den Ausschnitten 8 der Bleche 6 in Verbindung.

Im Betrieb wird nach dem Einführen und Tiefenpositionieren des Verankerungselements über die Bohrung 16 und die Bohrungen 17 entweder Knochenzement zur zusätzlichen Verankerung, der durch die Einschnitte 8 austritt und aushärtet, eingeführt oder ein Wirkstoff wie z.B. ein Arzneimittel oder eine wachstumsfördernde Substanz dem umliegenden Knochenbereich zugeführt.

Die radialen Bohrungen 17 müssen in ihrer Anzahl nicht der Anzahl der Einschnitte 8 entsprechen, sondern können entsprechend weniger sein. Ferner kann die zentrale Bohrung 16 auch durch die Spitze 3 hindurchgehen, so daß auch am Ende der Spitze 3 ein Austritt der Substanz ermöglicht wird.

Fig. 11b) und 11a) zeigen beispielhaft alternative Querschnittsformen des Knochenverankerungselements mit ebenen oder konkaven Seitenwänden. Die Querschnittsform ist nicht darauf beschränkt.

In einer in Fig. 12 gezeigten weiteren Abwandlung sind die radialen Bohrungen 17 nicht in Umfangsrichtung zu den Ausschnitten 8 der Bleche 6 hin gerichtet, sondern münden in die Wandabschnitte 18 des Schafts, in denen keine Bleche 6 vorgesehen sind.

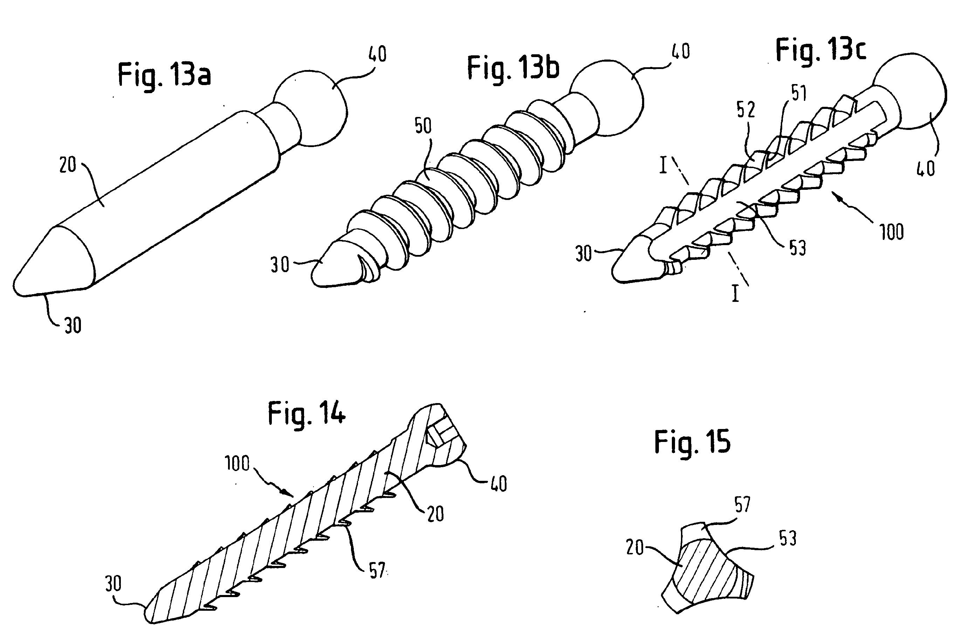

Die Figuren 13a) bis 13c) zeigen Herstellungsschritte eines Verankerungselements 100 nach einer zweiten Ausführungsform, und Fig. 14 zeigt einen Querschnitt in Längsrichtung des Verankerungselements nach der zweiten Ausführungsform, welches in Fig. 13c) gezeigt ist.

Wie in Fig. 13a) ersichtlich ist, wird im ersten Schritt der Herstellung des Verankerungselements 100 nach der zweiten Ausführungsform ein Element mit einem Schaft 20, einer Spitze 30 an einem Ende des Schaftes und einem Kopf 40 am gegenüberliegenden Ende bereitgestellt. In dem in Fig. 13b) gezeigten zweiten Schritt wird in den Schaft 20 ein Gewinde 50 geschnitten. In einem nächsten Schritt wird in dem Gewinde 50 eine Hinterschneidung derart erzeugt, daß, wie aus Fig. 13c) ersichtlich ist, die dem Kopf 40 zugewandte Teilflanke 51 mit der der Spitze 30 zugewandten Teilflanke 52 einen Winkel einschließt, der nur wenige Grad beträgt. Dadurch ist eine gewisse Elastizität erzeugt. Wie aus Fig. 13c) ersichtlich ist, werden anschließend in Längsrichtung des Schafts Abschnitte 53 herausgefräst, die vorzugsweise zur Mittelachse des Schafts hin konkav ausgebildet sind und die sich über die gesamte Schaftlänge erstrecken. Es werden wenigstens zwei, bevorzugt aber mehrere gleichmäßig in Umfangsrichtung beabstandete Abschnitte 53 gebildet. Dadurch entstehen mit den verbleibenden Gewindeteilen mit Hinterschneidung Widerhakenelemente 57, ähnlich den Widerhakenelementen 7 der ersten Ausführungsform.

Das Verankerungselement nach dieser Ausführungsform ist beispielsweise aus einem körperfreundlichen Kunststoffmaterial, z.B. einem hochmolekularem Polyethylen gebildet. Dadurch läßt sich am einfachsten die erforderliche Elastizität der Widerhakenelemente 57 erzielen.

Weitere Abwandlungen der beschriebenen Ausführungsformen sind möglich und Elemente der einzelnen Ausführungsformen und Abwandlungen derselben können miteinander kombiniert werden.

Beispielsweise hat in einer weiteren Abwandlung das Verankerungselement 100 nach der zweiten Ausführungsform ebenfalls eine koaxiale Bohrung und sich davon in radialer Richtung in die Wand erstreckende Bohrungen zum Einführen einer Wirksubstanz.
Die Widerhakenelemente müssen nicht trapezförmig sein, sondern können auch quadratisch, rechteckig oder anderweitig geformt sein.

Anders als bei dem in Fig. 1 gezeigten Ausführungsbeispiel können die Widerhakenelemente einer axialen Reihe in gleichen Abständen in axialer Richtung vorgesehen sein, so daß an der Stelle jeder Windung ein Widerhakenelement vorhanden ist. Alternativ können die Abstände der Widerhakenelemente einer Reihe in axialer Richtung variieren, wobei aber der Abstand jeweils ein ganzes Vielfaches eines kleinsten Abstands zweier Widerhakenelemente einer axialen Reihe ist. Durch unterschiedliche Abstände können unterschiedliche Knochendichten berücksichtigt werden und die Belastung auf bestimmte Knochenstrukturen reduziert werden.

In einer weiteren Abwandlung sind die Widerhakenelemente aus einer Formgedächtnislegierung derart gebildet, daß sie bei Körpertemperatur abstehen und bei einer niedrigeren Temperatur, z.B. bei Raumtemperatur, anliegen.
Im Betrieb wird das Verankerungselement, bei dem die Widerhakenelemente anliegen, in die Kernlochbohrung eingedrückt. Nachdem es die Körpertemperatur angenommen hat, stellen sich die Widerhakenelemente auf.

Als Material für das Verankerungselement nach der ersten Ausführungsform, bzw. der Bleche mit den Widerhakenelementen, oder der einstückig ausgebildeten Schraube nach der zweiten Ausführungsform kann auch ein Normalmetall mit entsprechend hoher Elastizität verwendet werden.
Weiterhin ist die Verwendung eines resorbierbaren Materials möglich. Körperresorbierbare Kunststoffe, z.B. Polylactid (PLLA) sind denkbar.

In einer weiteren Abwandlung kann die Spitze 3 getrennt ausgebildet sein und mit dem Schaft verbindbar sein. Z.B. kann die Spitze an den Schaft 2 geschraubt werden, der hierzu an seinem der Spitze zugewandten Ende eine Bohrung mit Innengewinde aufweist und die Spitze einen darin einschraubbaren Abschnitt mit Außengewinde. Die Spitze kann auch an den Schaft geschweißt werden oder mit Übermaß gesteckt werden.

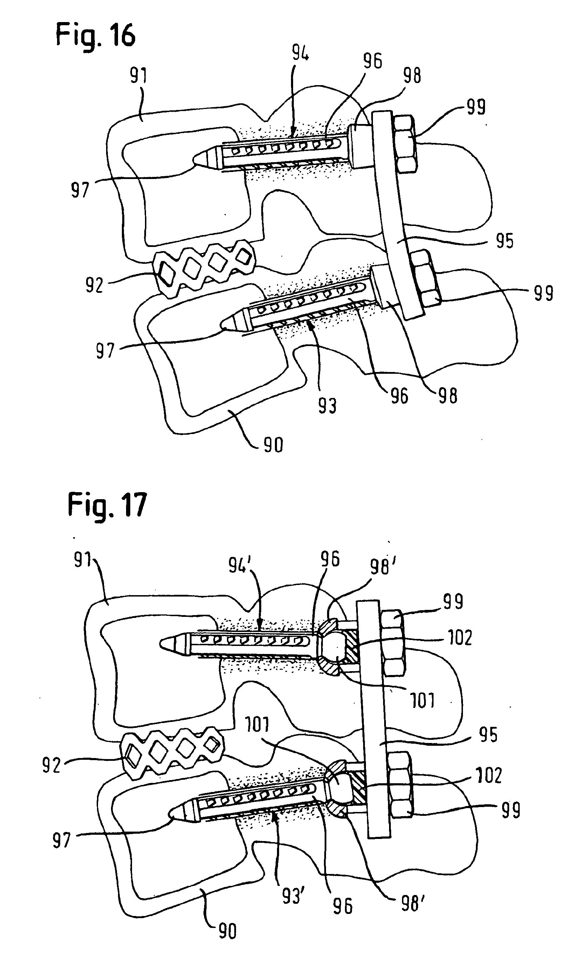

Fig. 16 zeigt ein erstes Anwendungsbeispiel, bei dem zum Zwecke der Versteifung zweier Wirbel 90, 91 unter Verwendung eines Fusionselements 92 Monoaxialverankerungselemente 93, 94 vorgesehen sind, über die ein Stab 95 im Wirbel verankert ist. Die Monoaxialschrauben 93, 94 weisen jede einen Schaft 96 mit einer Spitze 97 auf, wobei der Schaft nach einer der zuvor beschriebenen Ausführungsformen des Verankerungselements ausgebildet ist. An dem der Spitze 97 gegenüberliegenden Ende des Schafts 96 ist ein mit dem Schaft starr verbundenes Aufnahmeteil 98 vorgesehen, in welches in bekannter Weise der Stab 95 eingelegt ist und über ein Fixierelement 99, beispielsweise über eine auf das Aufnahmeteil aufschraubbare Mutter, gesichert ist. Bei der Monoaxialschraube ist die Ausbildung des Schafts 96 mit den aus der Formgedächtnislegierung gefertigten Blechen mit den Widerhakenelementen vorteilhaft, da nach dem Eindrücken in die Kernlochbohrung und vor dem Erwärmen des Verankerungselements das Verankerungselement in der Kernlochbohrung drehbar ist und so das Aufnahmeteil 98 relativ zu dem einzulegenden Stab ausrichtbar ist.

Fig. 17 zeigt ein weiteres Anwendungsbeispiel, welches sich von dem Anwendungsbeispiel gemäß Fig. 16 dadurch unterscheidet, daß anstelle der Monoaxial-Verankerungselemente Polyaxial-Verankerungselemente vorgesehen sind. Die Polyaxial-Verankerungselemente 93', 94' weisen einen Schaft 96 mit einer Spitze 97 auf, wobei der Schaft 96 nach einer der Ausführungsformen des erfindungsgemäßen Verankerungselements mit den Widerhakenelementen ausgebildet ist. An dem der Spitze 97 gegenüberliegenden Ende des Schafts 96 ist ein Kopf 101 vorgesehen, der in einem Aufnahmeteil 98' in nichtfixiertem Zustand schwenkbar gehalten ist. In das Aufnahmeteil ist in bekannter Weise ein Stab 95 einlegbar, der über ein Druckstück 102 oder (nicht dargestellt) direkt auf den Kopf 101 drückt und somit die Schwenkstellung des Kopfes relativ zu dem Aufnahmeteil fixiert.

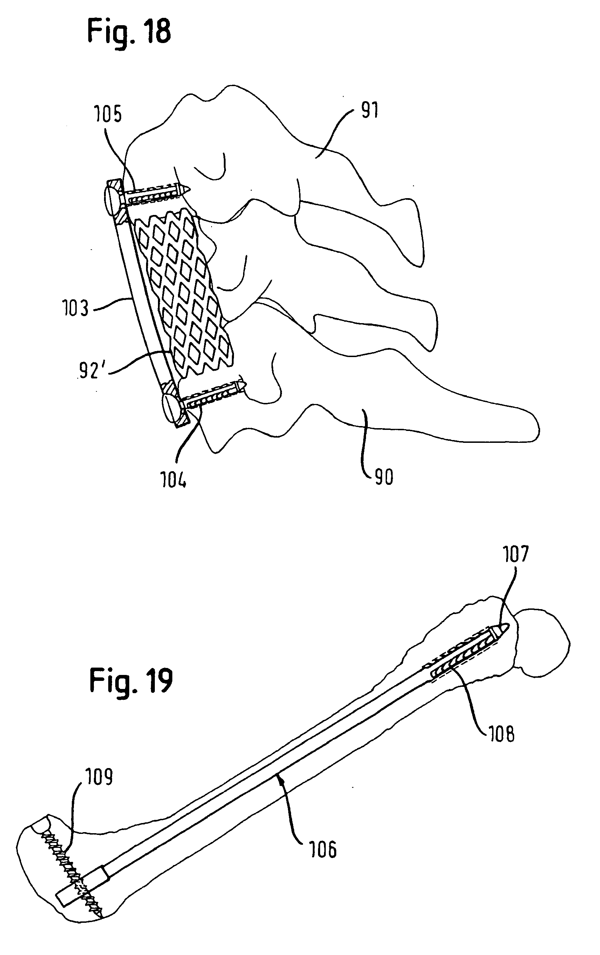

Fig. 18 zeigt als weiteres Anwendungsbeispiel die Versteifung zweier Wirbelknochen 90, 91 nach Entfernung eines dazwischenliegenden Wirbelteiles über ein Fusionselement 92' und eine Platte 103, die über Verankerungselemente 104, 105 in den Wirbeln verankert ist. Die Verankerungselemente 104, 105 weisen einen Schaft mit Widerhakenelementen gemäß der Erfindung auf und einen Kopf, über den die Platte 103 gegen den Wirbelknochen gedrückt wird. Das Verankerungselement ist auch zusammen mit in der Unfallchirurgie verwendeten Platten einsetzbar.

Fig. 19 zeigt als weiteres Anwendungsbeispiel einen Marknagel 106, der einen Schaft und eine Spitze 107 aufweist. Angrenzend an die Spitze weist der Schaft einen Abschnitt 108 auf, der wie bei dem erfindungsgemäßen Verankerungselement mit Widerhaken ausgestattet ist. Der Marknagel wird in einen Röhrenknochen eingeführt und über eine Schraube 109 gesichert.

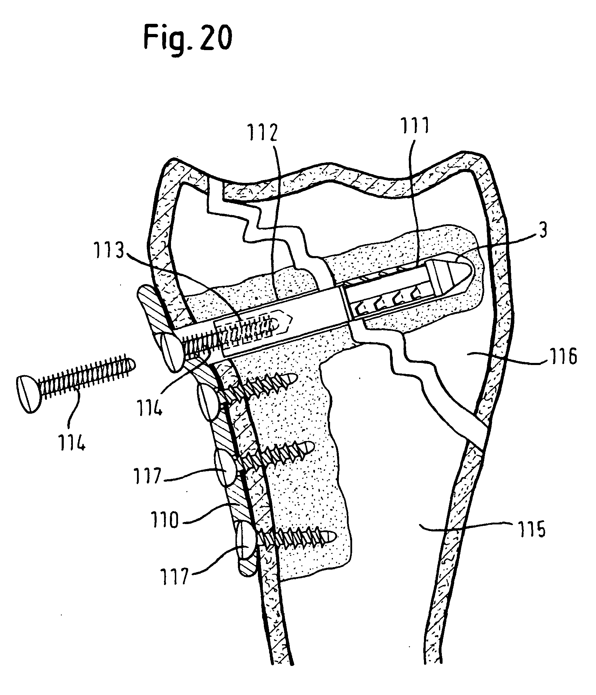

Fig. 20 zeigt ein weiteres Ausführungsbeispiel, bei dem das Knochenverankerungselement zusammen mit einer Platte 110 zur Fixierung einer Knochenfraktur eingesetzt wird. Das Knochenverankerungselement weist einen ersten an die Spitze 3 angrenzenden Schaftabschnitt 111 auf, der die Widerhakenelemente beinhaltet und angrenzend daran einen zweiten Schaftabschnitt 112 mit einer von dem der Spitze 3 gegenüberliegenden Ende sich ins Innere erstreckende Sacklochbohrung 113 mit einem Innengewinde. Zum Verbinden des Knochenverankerungselements mit der Platte 110 ist eine in den Abschnitt mit dem Innengewinde des Verankerungselements einschraubbare Schraube 114 vorgesehen. Im Betrieb wird nach Erzeugen der Kernlochbohrung in den zu fixierenden Knochenfragmenten 115, 116 das Verankerungselement eingepreßt. Die Kernlochbohrung ist dabei von solch einer Länge, daß der Abschnitt 111 mit den Widerhakenelementen und die Spitze 3 des Verankerungselements in dem einen Knochenfragment 116 positionierbar sind und der Schaftabschnitt 112 mit dem Innengewinde in dem anderen Knochenfragment 115. Beim Einschrauben der Schraube 114 in die Bohrung 113 mit dem Innengewinde werden aufgrund der Widerhakenfunktion des Abschnitts 111, der in dem einen Knochenfragment 116 liegt, die beiden Knochenfragmente 115 und 116 zueinander hingezogen. Weitere Schrauben 117 können zum Fixieren der Platte vorgesehen sein.

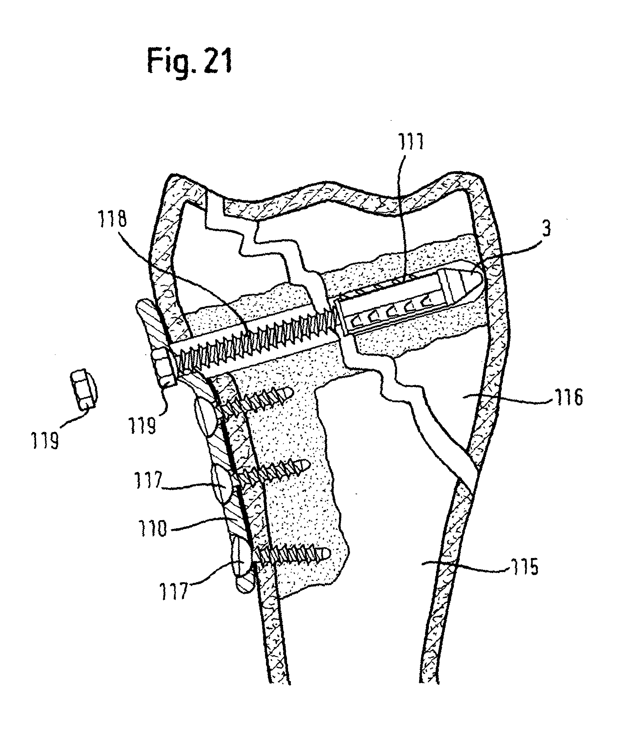

Das in Fig. 21 gezeigte Beispiel unterscheidet sich von dem in Fig. 20 gezeigten Ausführungsbeispiel des Verankerungselements dadurch, daß anstelle des Schaftabschnitts 112 mit der Bohrung 113 mit dem Innengewinde ein an den Abschnitt 111 mit den Widerhakenelementen angrenzender Schaftabschnitt 118 mit einem Außengewinde vorgesehen ist, auf das eine Mutter 119 aufschraubbar ist. Der Betrieb erfolgt wie bei dem in Fig. 20 beschriebenen Ausführungsbeispiel, nur daß anstelle des Einschraubens der Schraube 114 ein Aufschrauben der Mutter 119 stattfindet.

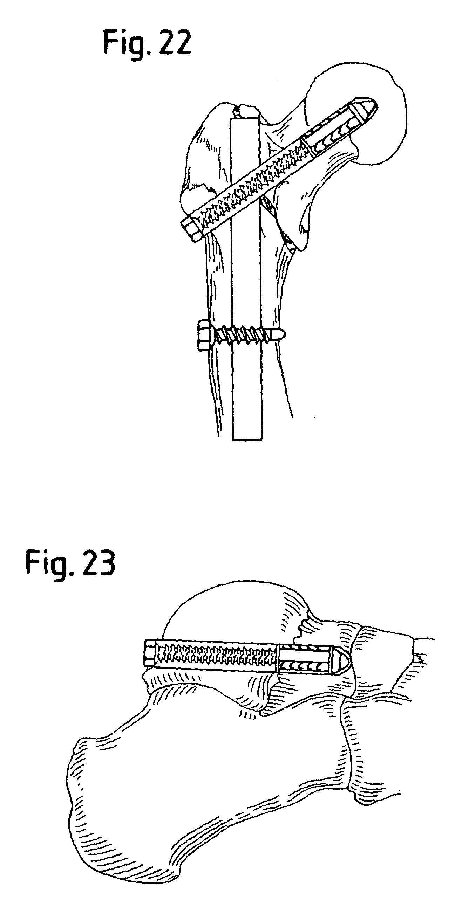

Fig. 22 zeigt ein Anwendungsbeispiel an einem Hüftknochen, Fig. 23 am Rückfuß.

## Patentansprüche

1. Knochenverankerungselement mit einem Schaft (2; 20) zum Verankern in einem Knochen (15), wobei
der Schaft einen Abschnitt mit einer Mehrzahl von in einer Schraubenlinie (S) um die Schaftachse (M) angeordneten Widerhakenelementen (7; 70; 57) aufweist,
**dadurch gekennzeichnet, daß** die Widerhakenelemente (7; 70; 57) relativ zum Schaftkörper elastisch bewegbar sind.

2. Knochenverankerungselement nach Anspruch 1, **dadurch gekennzeichnet, daß** die Widerhakenelemente (7; 70; 57) an ihrem freien Ende jeweils auf der Schraubenlinie (S) liegende Schneidkanten (10) haben.

3. Knochenverankerungselement nach Anspruch 1 oder 2, **dadurch gekennzeichnet, daß** die Widerhakenelemente (7; 70; 57) als im wesentlichen flache Trapeze ausgebildet sind, deren Basis (9) mit dem Schaft (2; 20) verbunden ist.

4. Knochenverankerungselement nach einem der Ansprüche 1 bis 3, **dadurch gekennzeichnet, daß** die Widerhakenelemente (7; 70; 57) in axialer Richtung des Schafts gesehen in wenigstens zwei axialen Reihen angeordnet sind.

5. Knochenverankerungselement nach einem der Ansprüche 1 bis 4, **dadurch gekennzeichnet, daß** sich die Abstände der Widerhakenelemente (7; 70) in axialer Richtung ändern.

6. Knochenverankerungselement nach einem der Ansprüche 1 bis 5, **dadurch gekennzeichnet, daß** wenigstens ein Teil des die Widerhakenelemente (70) enthaltenden Schaftabschnitts (60) aus einer Formgedächtnislegierung, bevorzugt aus Nitinol, gebildet ist.

7. Knochenverankerungselement nach einem der Ansprüche 1 bis 6, **dadurch gekennzeichnet, daß** wenigstens ein Teil des die Widerhakenelemente enthaltenden Schaftabschnitts aus einem körperverträglichen Kunststoff gebildet ist.

8. Knochenverankerungselement nach einem der Ansprüche 1 bis 7, **dadurch gekennzeichnet, daß** die Widerhakenelemente (7; 70) auf länglichen Blechen (6; 60) ausgebildet sind, die in in axialer Richtung des Schafts (2) verlaufende Nuten (5) eingeschoben sind.

9. Knochenverankerungselement nach Anspruch 8, **dadurch gekennzeichnet, daß** die Widerhakenelemente (7; 70) durch von der dem Schaftkörper abgewandten Oberfläche unter einem Winkel nach außen abstehende Ausschnitte der Bleche (6; 60) gebildet sind.

10. Knochenverankerungselement nach Anspruch 8 oder 9, **dadurch gekennzeichnet, daß** eine Sicherungsvorrichtung (12) vorgesehen ist, zum Sichern der Bleche (6; 60) in den Nuten (5).

11. Knochenverankerungselement nach einem der Ansprüche 8 bis 10, **dadurch gekennzeichnet, daß** die Bleche (60) mit den Widerhakenelementen (70) aus einer Formgedächtnislegierung gebildet sind.

12. Knochenverankerungselement nach einem der Ansprüche 1 bis 7, **dadurch gekennzeichnet, daß** der Schaft (20) und die Widerhakenelemente (57) einstückig ausgebildet sind.

13. Knochenverankerungselement nach einem der Ansprüche 1 bis 12, **dadurch gekennzeichnet, daß** der Schaft (2; 20) einen Kanal (16) aufweist.

14. Knochenverankerungselement nach Anspruch 13, **dadurch gekennzeichnet, daß** der Schaft in seiner Wandung Öffnungen (80; 17) aufweist, die mit dem Kanal (16) in Verbindung stehen.

15. Knochenverankerungselement nach einem der Ansprüche 1 bis 14, **dadurch gekennzeichnet, daß** das Knochenverankerungselement einen Kopf aufweist, der mit dem Schaft starr verbunden ist und zur Aufnahme eines Stabs ausgebildet ist.

16. Knochenverankerungselement nach einem der Ansprüche 1 bis 14, **dadurch gekennzeichnet, daß** das Knochenverankerungselement einen Kopf aufweist, der mit einem Aufnahmeteil zum Aufnehmen eines Stabes schwenkbar verbunden ist.

17. Knochenverankerungselement nach einem der Ansprüche 1 bis 14, **dadurch gekennzeichnet, daß** das Knochenverankerungselement ein Marknagel ist.

18. Knochenverankerungselement nach einem der Ansprüche 1 bis 14, **dadurch gekennzeichnet, daß** angrenzend an den die Widerhakenelemente beinhaltenden Schaftabschnitt (111) ein Schaftabschnitt (112; 118) mit einer sich von seinem freien Ende erstreckenden Bohrung (113) mit einem Innengewinde zum Einschrauben einer Schraube oder ein Schaftabschnitt (118) mit einem Außengewinde zum Aufschrauben eines Mutterelements vorgesehen ist.

19. Knochenverankerungselement nach einem der Ansprüche 1 bis 18, **dadurch gekennzeichnet, daß** die Widerhakenelemente jeweils eine mit dem Schaftkörper verbundene Basis (9) und ein freies Ende (10) aufweisen.

## Claims

1. Bone anchoring element with a shaft (2; 20) for anchoring in a bone (15) the shaft comprising a section with a plurality of barb elements (7; 70; 57) that are arranged on a helical line (S) around the shaft axis (M), **characterized in that** the barb elements (7; 70; 57) are elastically movable relative to the body of the shaft.

2. Bone anchoring element according to Claim 1, **characterized in that** the barb elements (7; 70; 57) each comprise on their free end cutting edges (10) which are positioned on the helical line (S).

3. Bone anchoring element according to Claim 1 or 2, **characterized in that** the barb elements (7; 70; 57) are provided as essentially flat trapezoids whose base (9) is connected to the shaft (2; 20).

4. Bone anchoring element according to any one of the Claims 1 to 3, **characterized in that** the barb elements (7; 70; 57), viewed in the direction of the shaft axis, are arranged in at least two axial rows.

5. Bone anchoring element according to any one of the Claims 1 to 4, **characterized in that** the distances between the barb elements (7; 70) in axial direction change.

6. Bone anchoring element according to any one of the Claims 1 to 5, **characterized in that** at least a part of the shaft section (60) containing the barb elements (70) is made from a shape memory alloy, preferably from nitinol.

7. Bone anchoring element according to any one of the Claims 1 to 6, **characterized in that** at least a part of the shaft section containing the barb elements is made from a body-compatible plastic material.

8. Bone anchoring element according to any one of the Claims 1 to 7, **characterized in that** the barb elements (7; 70) are provided on oblong metal plates (6; 60) which are slid into grooves (5) extending along the direction of the shaft (2) axis.

9. Bone anchoring element according to Claim 8, **characterized in that** the barb elements (7; 70) are formed by cuts of the metal plates (6; 60) which project outward by an angle from the surface facing away from the shaft body.

10. Bone anchoring element according to Claim 8 or 9, **characterized in that** a securing device (12) is provided for securing the metal plates (6; 60) in the grooves (5).

11. Bone anchoring element according to any one of the Claims 8 to 10, **characterized in that** the metal plates (60) with barb elements (70) are made from a shape memory alloy.

12. Bone anchoring element according to any one of the Claims 1 to 7, **characterized in that** the shaft (20) and the barb elements (57) are provided in one piece.

13. Bone anchoring element according to any one of the Claims 1 to 12, **characterized in that** the shaft (2; 20) comprises a channel (16).

14. Bone anchoring element according to Claim 13, **characterized in that** the wall of the shaft comprises orifices (80; 17) which are connected to the channel (16).

15. Bone anchoring element according to any one of the Claims 1 to 14, **characterized in that** the bone anchoring element comprises a head that is rigidly connected to the shaft and is provided to receive a rod.

16. Bone anchoring element according to any one of the Claims 1 to 14, **characterized in that** the bone anchoring element comprises a head that is connected in a pivoting fashion with a receiver member for receiving a rod.

17. Bone anchoring element according to any one of the Claims 1 to 14, **characterized in that** the bone anchoring element is an intermedullary nail.

18. Bone anchoring element according to any one of the Claims 1 to 14, **characterized in that**, adjacent to the shaft section (111) containing the barb elements, a shaft section (112; 118) with a bore hole (113), which extends from the section's free end and bears an internal thread for screwing-in a screw, or a shaft section (118) with an external thread for screwing-on a nut element is provided.

19. Bone anchoring element according to any one of the Claims 1 to 18, **characterized in that** the barb elements each comprise a base (9), connected to the shaft body, and a free end (10).

## Revendications

1. Elément d'ancrage osseux, comprenant une tige (2 ; 20) pour ancrage dans un os (15), où
la tige présente un tronçon comprenant une pluralité d'éléments formant contre-crochets (7 ; 70; 57) disposés selon une hélicoïde (S) autour de l'axe de tige (M),
**caractérisé en ce que** les éléments formant contre-crochets (7 ; 70 ; 57) sont déplaçables élastiquement par rapport au corps de tige.

2. Elément d'ancrage osseux selon la revendication 1, **caractérisé en ce que** les éléments formant contre-crochets (7 ; 70 ; 57) ont, à leur extrémité libre, chacun des arêtes de coupe (10) situées sur l'hélicoïde (S).

3. Elément d'ancrage osseux selon la revendication 1 ou 2, **caractérisé en ce que** les éléments formant contre-crochets (7 ; 70 ; 57) sont réalisés sous forme de trapèzes sensiblement plats, dont la base (9) est reliée à la tige (2 ; 20).

4. Elément d'ancrage osseux selon l'une des revendications 1 à 3, **caractérisé en ce que** les éléments formant contre-crochets (7 ; 70 ; 57) sont disposés, en observant dans la direction axiale de la tige, en au moins trois rangées axiales.

5. Elément d'ancrage osseux selon l'une des revendications 1 à 4, **caractérisé en ce que** les espacements des éléments formant contre-crochets (7 ; 70) varient en évoluant dans la direction axiale.

6. Elément d'ancrage osseux selon l'une des revendications 1 à 5, **caractérisé en ce qu'**au moins une partie du tronçon de tige (60) contenant les éléments formant contre-crochets (70) est formée d'un alliage à mémoire de forme, de préférence en Nitinol.

7. Elément d'ancrage osseux selon l'une des revendications 1 à 6, **caractérisé en ce qu'**au moins une partie du tronçon de tige contenant les éléments formant contre-crochets est formée d'une matière synthétique compatible avec le corps.

8. Elément d'ancrage osseux selon l'une des revendications 1 à 7, **caractérisé en ce que** les éléments formant contre-crochets (7 ; 70) sont réalisés sur des tôles (6 ; 60) allongées, insérées dans des rainures (5) s'étendant dans la direction axiale de la tige (2).

9. Elément d'ancrage osseux selon la revendication 8, **caractérisé en ce que** les éléments formant contre-crochets (7 ; 70) sont formés par des découpures des tôles (6 ; 60), faisant saillie vers l'extérieur sous un certain angle vis-à-vis de la surface opposée au corps de tige.

10. Elément d'ancrage osseux selon la revendication 8 ou 9, **caractérisé en ce qu'**un dispositif de sécurité (12) est prévu pour assurer les tôles (6 ; 60) dans les nervures (5).

11. Elément d'ancrage osseux selon l'une des revendications 8 à 10, **caractérisé en ce que** les tôles (60) avec les éléments formant contre-crochets (70) sont formées d'un alliage à mémoire de forme.

12. Elément d'ancrage osseux selon l'une des revendications 1 à 7, **caractérisé en ce que** la tige (20) et les éléments formant contre-crochets (57) sont formés d'une seule pièce.

13. Elément d'ancrage osseux selon l'une des revendications 1 à 12, **caractérisé en ce que** la tige (2 ; 20) présente un canal (16).

14. Elément d'ancrage osseux selon la revendication 13, **caractérisé en ce que** la tige présente dans sa paroi des ouvertures (80 ; 17) reliées au canal (16).

15. Elément d'ancrage osseux selon l'une des revendications 1 à 14, **caractérisé en ce que** l'élément d'ancrage osseux présente une tête, reliée rigidement à la tige et conformée pour recevoir une barre.

16. Elément d'ancrage osseux selon l'une des revendications 1 à 14, **caractérisé en ce que** l'élément d'ancrage osseux présente une tête, réalisée de façon à pouvoir pivoter avec une partie de logement pour recevoir une barre.

17. Elément d'ancrage osseux selon l'une des revendications 1 à 14, **caractérisé en ce que** l'élément d'ancrage osseux est un clou d'ostéosynthèse intra-médullaire.

18. Elément d'ancrage osseux selon l'une des revendications 1 à 14, **caractérisé en ce qu'**en limite au tronçon de tige (111) contenant les éléments formant contre-crochets est prévu un tronçon de tige (112 ; 118) avec un perçage (113) s'étendant depuis son extrémité libre, avec un filetage intérieur pour le vissage d'une vis, ou un tronçon de tige (118) avec un filetage extérieur pour rapporter par vissage un élément formant écrou.

19. Elément d'ancrage osseux selon l'une des revendications 1 à 18, **caractérisé en ce que** les éléments formant contre-crochets présentent chacun une base (9) reliée au corps de tige, et une extrémité (10) libre.
